(19) Europäisches Patentamt
European Patent Office
Office européen des brevets

(11) EP 2 111 860 A1

(12) EUROPEAN PATENT APPLICATION
published in accordance with Art. 153(4) EPC

(43) Date of publication:
28.10.2009 Bulletin 2009/44

(21) Application number: 05753288.9

(22) Date of filing: 15.06.2005

(51) Int Cl.:
*A61K 31/315* (2006.01) *A61K 31/198* (2006.01)
*A61K 31/20* (2006.01) *A61P 17/16* (2006.01)

(86) International application number:
PCT/JP2005/011404

(87) International publication number:
WO 2005/123062 (29.12.2005 Gazette 2005/52)

(84) Designated Contracting States:
AT BE BG CH CY CZ DE DK EE ES FI FR GB GR HU IE IS IT LI LT LU MC NL PL PT RO SE SI SK TR

(30) Priority: 18.06.2004 JP 2004180880

(71) Applicant: Ajinomoto Co., Inc.
Tokyo 104-8315 (JP)

(72) Inventors:
- IWASAKI, Keiji,
AJINOMOTO CO., INC.
Kawasaki-shi, Kanagawa 210-8681 (JP)
- KITAZAWA, Manabu,
AJINOMOTO CO., INC.
Kawasaki-shi, Kanagawa 210-8681 (JP)
- TAKINO, Yoshinobu,
AJINOMOTO CO., INC.
Kawasaki-shi, Kanagawa 210-8681 (JP)

(74) Representative: Strehl Schübel-Hopf & Partner
Maximilianstrasse 54
80538 München (DE)

# (54) INFLAMMATION INHIBITOR COMPRISING ZINC SALT OF ACYLAMINO ACID

(57) An object is to provide an inflammation inhibitor which gives a good feeling of use.

The inflammation inhibitor for the skin **characterized by** comprising a zinc salt of an acylamino acid is provided and further, a cosmetic **characterized by** containing the inflammation inhibitor is provided.



Printed by Jouve, 75001 PARIS (FR)

## Description

### Technical Field

The present invention relates to an inflammation inhibitor, which is useful for preventing, delaying, improving or treating an inflammatory disease or skin damage or disease caused by inflammation, or a cosmetic and a skin preparation for external use containing such an inflammation inhibitor as an active ingredient.

### Background Art

As a factor causing inflammation of the skin, there are infectious substances such as bacteria and viruses, antigenic substances causing allergic reactions, stimulating factors causing tissue damage, ultraviolet light inducing skin cancer and accelerating skin aging and the like. Due to these factors, the skin receives various damage, therefore, inhibition of the excessive inflammatory reaction which is a cause thereof leads to alleviation of the skin damage.

In recent years, a study on the mechanism of action of inflammation of the skin has progressed, and it has been revealed that an inflammatory cytokine such as IL-1$\alpha$ or TNF-$\alpha$, or an extracellular matrix metalloproteinase such as collagenase is involved as the factor. The expression of such a cytokine or extracellular matrix metalloproteinase is regulated at the gene level by a transcriptional regulator such as NF-$\kappa$B or AP-1. For example, it has been reported that when the skin is exposed to ultraviolet light included in the sunlight, NF-$\kappa$B or AP-1 in a skin cell is activated, which is a cause of accelerating skin aging (for example, Non-patent document 1). Accordingly, it is expected that if these inflammatory factors can be inhibited, skin disorders caused by various factors can be alleviated.

As a substance inhibiting an inflammatory factor causing skin damage, various substances have been reported. For example, it is disclosed that an antioxidant substance such as N-acetyl-L-cysteine inhibits the activation of NF-$\kappa$B or AP-1 in an epidermal cell (for example, Non-patent documents 2 and 3). However, there are problems that its effective concentration is 10 mM to 30 mM, and the degree of the effect is not sufficient or the toxicity to a cell is strong and the like. Other than N-acetyl-L-cysteine, it has been reported that retinoic acid inhibits the activation of AP-1 and the expression of an extracellular matrix metalloproteinase (for example, Non-patent document 1). However, retinoic acid has side effects such as stimulation and peeling of the skin, and its use is limited. On the other hand, as the damage caused by inflammation of the skin, skin damage caused by ultraviolet light can be exemplified, however, it is known that this skin disorder is inhibited by induction of metallothionein, which is an endogenous antioxidant substance in the skin, due to a zinc salt of an amino acid or a zinc salt of a fatty acid (for example, Patent documents 1 and 2). However, the effects of these compounds are not sufficient, and also it has not been reported that the activation of a gene transcription factor such as AP-1, which is a major factor of inflammation caused by ultraviolet light, is inhibited by these compounds. Further, it has been reported that zinc oxide, which is a substance related to zinc, prevents diaper rash (for example, Non-patent document 4), however, its effect is not sufficient, and also it has not been reported that the activation of a gene transcription factor such as AP-1, which is a major factor of inflammation by ultraviolet light, is inhibited by this substance. On the other hand, it has been reported that there is a possibility that zinc acetate may prevent arteriosclerosis by inhibiting the activity of AP-1 that increases when vascular endothelial cells are cultured when zinc is lacking (Non-patent document 5), however, it is not known whether or not it inhibits inflammation of the skin.

[Patent document 1] International publication WO 00/44341
[Patent document 2] International publication WO 93/14748
[Non-patent document 1] Nature, Vol. 379, pp. 335-339, 1996
[Non-patent document 2] Free Rad. Biol. Med. Vol. 26, pp. 174-183
[Non-patent document 3] FEBS Letters, Vol. 384, pp. 92-96, 1996
[Non-patent document 4] Eur. Acad. Dermatol. Veneol. 15 (Suppl. 1) pp. 5-11, 2001
[Non-patent document 5] J Am. Clloge Nutrt. 16(5), pp. 411-417, 1997

### Disclosure of the invention

In view of the above background art, an object of the present invention is to provide a cosmetic or a skin preparation for external use containing as an active ingredient an inflammation inhibitory component which prevents, improves or treats inflammatory disorder of the skin by inhibiting the activation of a gene transcription factor of an extracellular matrix metalloproteinase.

The present inventors have made intensive studies in order to achieve the above object, and as a result, they found that a zinc salt of an acylamino acid represented by the following general formula (I) inhibits the activation of a gene transcription factor of an extracellular matrix metalloproteinase, thus the present invention has been completed.

That is, the present invention includes at least the following contents.

The present invention relates to an inflammation inhibitor characterized by comprising as an active ingredient, one or more kinds of zinc salts of an acylamino acid represented by the following general formula (I).

[Chemical 1]

$$\begin{array}{l} \qquad\quad\; R3 \\ \qquad\quad\;\; | \\ R1-N-CH(CH_2)_nCOOH \qquad (I) \\ \qquad\, | \\ \quad\;\; R2 \end{array}$$

(In the above general formula (I), R1 represents an acyl group having 2 to 22 carbon atoms, R2 represents a hydrogen atom or a linear or branched alkyl group having 1 to 6 carbon atoms, R3 represents an amino acid side chain of valine, leucine, isoleucine, phenylalanine, methionine, tryptophan, asparagine, glutamine, serine, tyrosine, aspartic acid, glutamic acid, lysine, arginine or histidine, or a hydrogen atom, and n represents an integer of 0 or 1.)

Further, in the case where R2 is a hydrogen atom, n is preferably 0, and an inflammation inhibitor characterized by comprising a zinc salt of an acylamino acid represented by the following general formula (II):

$$\begin{array}{l} \qquad\qquad\;\; R5 \\ \qquad\qquad\;\;\; | \\ R4-NH-CHCOOH \qquad (II) \end{array}$$

(in the above general formula (II), R4 represents an acyl group having 2 to 22 carbon atoms, R5 represents an amino acid side chain of valine, leucine, isoleucine, phenylalanine, methionine, tryptophan, asparagine, glutamine, serine, tyrosine, aspartic acid, glutamic acid, lysine, arginine or histidine, or a hydrogen atom) is also included in the present invention.

Another aspect of the present invention relates to a preventive or therapeutic agent for an inflammatory disease, characterized by comprising the above inflammation inhibitor. Further, the present invention relates to a cosmetic additive or a skin preparation for external use, characterized by comprising the above inflammation inhibitor. Still another aspect of the present invention is a method of preventing, delaying, improving or treating a skin disease, characterized by applying a skin preparation for external use comprising one or more agents of the above inflammation inhibitors as an active ingredient to the skin, and the skin disease may be a skin change due to aging or a skin change of which is not preferred cosmetically, those are accelerated by ultraviolet light.

Advantage of the Invention

According to the present invention, an inflammation inhibitor that can be preferably used for a cosmetic or the like can be obtained.

Best Mode for Carrying Out the Invention

Hereinafter, the present invention will be described in detail.

First, specific examples of the compound according to the present invention will be described. In an acylamino acid represented by the following general formula (I):

[Chemical 2]

$$\begin{array}{l} \qquad\qquad\;\; R3 \\ \qquad\qquad\;\; | \\ R1-N-CH(CH_2)_nCOOH \quad (I) \\ \qquad\;\; | \\ \qquad\;\; R2 \end{array}$$

examples of R1 include an acetyl group, a propioyl group, an isopropiol group, an n-butyloyl group, an isobutyloyl group, a sec-butyloyl group, a tert-butyloyl group, an n- amyloyl group, a sec-amyloyl group, a tert-amyloyl group, an isoamyloyl group, an n-hexyloyl group, a cyclohexyloyl group, an n-heptanoyl group, an n-octanoyl group, a 2-ethylhexyloyl group, a nonyoyl group, an isononyoyl group, a decanoyl group, an isodecanoyl group, an undecanoyl group, a lauroyl group, a tridecanoyl group, an isotridecanoyl group, a myristoyl group, a palmitoyl group, an isopalmitoyl group, a stearoyl group, an isostearoyl group, an oleoyl group, a docosanoyl group, a cocoyl group and the like. Among these, from the viewpoint of a feeling of use and an effect, an n-octanoyl group, a decanoyl group, a lauroyl group, a myristoyl group, a palmitoyl group, a stearoyl group, an oleoyl group, a cocoyl group and the like are preferred, and further, a lauroyl group, a myristoyl group, a palmitoyl group and a cocoyl group are particularly preferred. Examples of R2 include a methyl group, an ethyl group, a propyl group, an isopropyl group, an n-butyl group, an isobutyl group, a sec-butyl group, a tert-butyl group, an n-amyl group, a sec-amyl group, a tert-amyl group, an isoamyl group, an n-hexyl group, a cyclohexyl group, a hydrogen atom and the like. Among these, from the viewpoint of a feeling of use and an effect, a methyl group, an ethyl group, a propyl group, an isopropyl group and hydrogen atom are preferred, and particularly preferred are a methyl group and a hydrogen atom. Examples of R3 include a side chain of valine, leucine, isoleucine, phenylalanine, methionine, tryptophan, asparagine, glutamine, serine, tyrosine, aspartic acid, glutamic acid, lysine, arginine or histidine, which is an amino acid, a hydrogen atom and the like. Among these, a side chain of valine, leucine, isoleucine, serine, aspartic acid, glutamic acid or histidine and a hydrogen atom are preferred, and further, a side chain of glutamic acid or histidine and a hydrogen atom are particularly preferred. The tertiary structure of the amino acid moiety may be any of L-form, D-form and DL-form, however, it is preferably the L-form. n represents an integer of 0 or 1.

Here, as a preferred combination of substituents, when R1 is an n-octanoyl group, a decanoyl group, a lauroyl group, a myristoyl group, a palmitoyl group, a stearoyl group, an oleoyl group or a cocoyl group, R2 is a methyl group, an ethyl group, a propyl group, an isopropyl group or hydrogen atom, and R3 is a side chain of valine, leucine, isoleucine, serine, aspartic acid, glutamic acid or histidine or a hydrogen atom, and particularly preferably, R1 is a lauroyl group, R2 is a hydrogen atom, and R3 is a side chain of glutamic acid or histidine or a hydrogen atom, etc. can be exemplified. Further, when R2 is a hydrogen atom, n is preferably 0, and the compound is represented by the following general formula (II).

[Chemical 3]

$$\begin{array}{l} \qquad\qquad\quad R5 \\ \qquad\qquad\quad | \\ R4-NH-CHCOOH \quad (II) \end{array}$$

Here, the definition of R4 is the same as that of the above R1, and the definition of R5 is the same as that of the above R3. Incidentally, the present invention is not limited to these.

The inflammation inhibitor of the present invention can be orally or parenterally administered, however, it is preferably administered by applying it to the surface of the skin. In the case where a zinc salt of an acylamino acid represented by the above general formula (I) or a zinc salt of the above organic acid is blended in a cosmetic or a skin preparation for external use as an active ingredient for preventing or improving an inflammatory skin disease or inflammatory skin damage, the blending amount thereof can be set to 0.01% to 10% by weight, preferably 0.1% to 5% by weight. When the blending amount is less than 0.01% by weight, the ability of inhibiting inflammation is not sufficiently

exhibited, and when it exceeds 10%, a problem arises with respect to a feeling of use, for example, a scraping feeling against the skin is caused, therefore, either case is not preferred. When a zinc salt of an acylamino acid represented by the above general formula (I) is blended in a cosmetic or a skin preparation for external use, other than this component, a component or an excipient which is generally used in a cosmetic or a skin preparation for external use can be added in a range which does not inhibit the effect of the present invention.

Examples of the component which is generally used in a cosmetic or a skin preparation,for external use include an antioxidant, an anti-inflammatory agent, an ultraviolet absorber, a whitening agent, a cell activator, a moisturizing agent, a metal chelating agent, an oleaginous material, a surfactant, a solvent, a polymeric substance, a powdery substance, a dye, a fragrance, a transdermal absorption promoting agent, a steroid hormone and the like.

Examples of the antioxidant include the vitamin A group including retinol, dehydroretinol, retinol acetate, retinol palmitate, retinal, retinoic acid and vitamin A oil and derivatives thereof, carotenoids such as α-carotene, β-carotene, γ-carotene, cryptoxanthin, astaxanthin and fucoxanthin and derivatives thereof, the vitamin B group including pyridoxine, pyridoxal, pyridoxal-5-phosphate ester and pyridoxamine and derivatives thereof, the vitamin C group including ascorbic acid, sodium ascorbate, ascorbyl stearate, ascorbyl palmitate, ascorbyl dipalmitate and ascorbate magnesium phosphate and derivatives thereof, the vitamin D group including ergocalciferol, cholecalciferol and 1,25-dihydroxy-cholecalciferol and derivatives thereof, the vitamin E group including α-tocopherol, β-tocopherol, γ-tocopherol, δ-tocopherol, α-tocotrienol, β-tocotrienol, γ-tocotrienol, δ-tocotrienol, tocopherol acetate and tocopherol nicotinate and derivatives thereof, trolox and derivatives thereof, dihydroxytoluene, butylhydroxytoluene, butylhydroxyanisole, dibutylhydroxytoluene, α-lipoic acid, dehydrolipoic acid, glutathione and derivatives thereof, uric acid, erythorbic acids such as erythorbic acid and sodium erythorbate and derivatives thereof, gallic acid and gallic acid derivatives such as propyl gallate, rutin and rutin derivatives such as α-glycosyl-rutin, tryptophan and derivatives thereof, histidine and derivatives thereof, cysteine derivatives such as N-acetylcysteine, N-acetylhomocysteine, N-octanoylcysteine, and N-acetylcysteine methyl ester, cystine derivatives described in WO/0021925 such as N,N'-diacetylcystine dimethyl ester, N,N'-dioctanoylcystine dimethyl ester, and N,N'-dioctanoylhomocystine dimethyl ester, carnosine and derivatives thereof, homocamosine and derivatives thereof, anserine and derivatives thereof, carcinine and derivatives thereof, dipeptide or tripeptide derivatives including histidine and/or tryptophan and/or histamine, flavonoids such asflavanone, flavone, anthocyanin, anthocyanidine, flavonol, quercetin, quercitrin, myricetin, fisetin, hamamelitannin, catechin, epicatechin, gallocatechin, epigallocatechin, epicatechin gallate, and epigallocatechin gallate, tannic acid, caffeic acid, ferulic acid, protocatechuic acid, chalcone, oryzanol, carnosol, sesamol, sesamin, sesamolin, zingerone, curcumin, tetrahydrocurcumin, clovamide, deoxyclovamide, shogaol, capsaicin, vanillyl amide, ellagic acid, bromophenol, flavogracin, melanoidin, riboflavin, riboflavin butyrate ester, flavin mononucleotide, flavin adenine nucleotide, ubiquinone, ubiquinol, mannitol, bilirubin, cholesterol, ebselen, selenomethionine, ceruloplasmin, transferrin, lactoferrin, albumin, bilirubin, superoxide dismutase, catalase, glutathione peroxidase, metallothionein, o-phosphono-pyridoxylidene rhodamine, and N-(2-hydroxybenzyl)amino acid described in U.S. Pat. No. 5,594,012 and derivatives thereof, and N-(4-pyridoxylmethylene)amino acid and derivatives thereof and the like.

Examples of the anti-inflammatory agent include phenylbutazone, indomethacin, ibuprofen, ketoprofen, allantoin, guaiazulene, resorcin, hydrocortisone, prednisolone, methylprednisolone, dexamethasone, triamcinolone, triamcinolone acetonide, fludoxycortide, clobetasone, clobetasol and esters of these steroids, ketal, acetal and hemiacetal derivatives, flufenamic acid, bufexamac, naploxen, fluviprofen, fenbufen, tenoxicam, piroxicam, mefenamic acid, salicylic acid, salicylate derivatives such as sodium salicylate, methyl salicylate, and glycol salicylate, D-panthenol and derivatives thereof, glycyrrhizic acid and derivatives thereof such as methyl glycyrrhizinate, and dipotassium glycyrrhizinate, glycyrrhetinic acid and derivatives thereof such as glyceryl glycyrrhate, stearyl glycyrrhate and glycyrrhetinyl stearate, chondroitin sulfate, ε-aminocaproic acid, sodium diclofenac, tranexamic acid, diphenhydramine hydrochloride, chlorpheniramine maleate, ichthammol, γ-oryzanol, thianthol, sodium copper chlorophyllin, Angelica keiskei extract, Arnica Montana extract, aloe extract, Bistorta major extract, turmeric extract, Hypericum erectum extract, German chamomile extract, Glycyrrhiza glabra extract, Lonicera japonica extract, Nasturtium officinale extract, Symphytum officinale extract, Acanthopanax gracilistylus extract, salvia extract, Lithospermum root extract, Perilla extract, white birch extract, tea extract, Angelica acutiloba extract, Calendula officinalis extract, elderberry extract, Typha angustifolia extract, Sapindus mukurossi extract, Artemisia extract, eucalyptus extract, Astragalus sinicus extract, zinc oxide and the like.

Examples of the ultraviolet absorber include cinnamic acid-based ultraviolet absorbers such as p-methoxycinnamate-2-ethylhexyl, isopropyl p-methoxycinnamate, sodium p-methoxycinnamate, potassium p-methoxycinnamate, p-methoxycinnamate-2-ethoxyethyl, p-methoxyhydrocinnamate diethanolamine salt, di-p-methoxycinnamate-mono-2-ethylhexanoate glyceryl, octyl methoxycinnamate and methyl diisopropylcinnamate, benzophenone-based ultraviolet absorbers such as 2-hydroxy-4-methoxybenzophenone, 2-hydroxy-4-methoxybenzophenone-5-sulfuric acid, 2-hydroxy-4-methoxybenzophenone-5-sulfate sodium, dihydroxybenzophenone, 2,4-dihydroxybenzophenone, 2,2'-dihydroxy-4,4'-dimethoxybenzophenone, 2,2'-dihydroxy-4-methoxybenzophenone, dihydroxydimethoxybenzophenonesulfate sodium, 2,2',4,4'-tetrahydroxybenzophenone, and 2-hydroxy-4-n-octoxybenzophenone, benzoic acid-based ultraviolet absorbers such as p-aminobenzoic acid, sodium p-aminobenzoate, ethyl p-aminobenzoate, butyl p-aminobenzoate, p-dimethylaminobenzoate-2-ethylhexyl, amyl p-dimethylaminobenzoate, glyceryl p-aminobenzoate, and amyl p-aminobenzoate, salicylic acid-based ultraviolet absorbers such as salicylate-2-ethylhexyl, salicylate triethanolamine, homomenthyl salicylate, salicylate dipropylene glycol, methyl salicylate, salicylate ethylene glycol, phenyl salicylate, amyl salicylate, benzyl salicylate, isopropylbenzyl salicylate, myristyl salicylate, and potassium salicylate, dibenzoylmethane-based ultraviolet absorbers such as 4-tert-butyl-4'-methoxydibenzoylmethane, 4-isopropyldibenzoylmethane, 4-methoxydibenzoylmethane, and 4-tert-butyl-4'-hydroxydibenzoylmethane, urocanic acid-based ultraviolet absorbers such as urocanic acid, and ethyl urocanate, menthyl-O-aminobenzoate, 2-phenyl-benzimidazole-5-sulfuric acid, 2-phenyl-5-methylbenzoxazole, 3-(4-methylbenzylidene)camphor, 2-ethylhexyl-2-cyano-3,3'-diphenyl acrylate, 2-ethyl-2-cyano-3,3'-diphenyl acrylate, 2-(2'-hydroxy-5-methylphenyl)benzotriazole, methyl anthranilate, ethyl anthranilate, menthyl anthranilate, 2,4,6-tris[4-(2-ethylhexyloxycarbonyl)anilino]-1,3,5-triaz ine, 3,3'-(1,4-phenylenedimethylidene)bis(7,7-dimethyl-2-oxo-bi cyclo[2.2.1]heptane-1-methanesulfonic acid) (Mexoryl SX), titanium oxide, zirconium oxide, cerium oxide, zinc oxide and the like.

Examples of the whitening agent include tyrosinase inhibitors, endothelin antagonists, α-MSH inhibitors, glabridin, glabrene, liquiritin, isoliquiritin, ellagic acid and derivatives thereof, kojic acid and derivatives thereof, hydroquinone such as arbutin and derivatives thereof, cysteine and derivatives thereof, the vitamin C group including ascorbic acid, sodium ascorbate, ascorbyl stearate, ascorbyl palmitate, ascorbyl dipalmitate, and ascorbate magnesium phosphate and derivatives thereof, glutathione and derivatives thereof, resorcin and derivatives thereof, neoagarobiose, agarose oligosaccharide, asparagus extract, Althaea extract, Bistorta extract, Artemisiae Capillaris extract, Pisum bean extract, rose fruit extract, Scutellaria root extract, Ononis spinosa extract, seaweed extract, Pyracantha fortuneana extract, Glycyrrhiza glabra extract, raspberry extract, Sophora flavescens extract, unrefined sugar extract, Spatholobus suberectus Dunn extract, Acanthopanax gracilistylus extract, wheat germ extract, Asiasari Radix extract, crataegus extract, Cassia mimosoides extract, peony root extract, white lily extract, Inula britannica extract, Mori cortex extract, soybean extract, placenta extract, Aralia elata extract, tea extract, Angelica acutiloba extract, molasses extract, Rosa multiflora Thunb extract, Ampelopsis japonica Makino extract, grape seed extract, Fagus crenata extract, Flodemannita extract, hops extract, Rosa rugosa extract, Chaenomeles sinensis extract, Saxifraga stolonifera extract, Coix seed extract, Momordica grosvenori extract and the like.

Examples of the cell activator include nucleic acid-related substances such as deoxyribonucleic acids, adenylic acid, ribonucleic acids, cyclic AMP, cyclic GMP, flavin adenine nucleotide, guanine, adenine, cytosine, thymine, xanthine, caffeine, and theophylline and derivatives thereof, the vitamin A group including retinol, dehydroretinol, retinol acetate, retinol palmitate, retinal, retinoic acid and vitamin A oil and derivatives thereof, carotenoids such as α-carotene, β-carotene, γ-carotene, cryptoxanthin, astaxanthin and fucoxanthin, and derivatives thereof, the vitamin B group including pyridoxine, pyridoxal, pyridoxal-5-phosphate ester and pyridoxamine and derivatives thereof, the vitamin C group including ascorbic acid, sodium ascorbate, ascorbyl stearate, ascorbyl palmitate, ascorbyl dipalmitate and ascorbate magnesium phosphate and derivatives thereof, the vitamin D group including ergocalciferol, cholecalciferol and 1,25-dihydroxy-cholecalciferol and derivatives thereof, the vitamin E group including α-tocopherol, β-tocopherol, γ-tocopherol, δ-tocopherol, α-tocotrienol, β-tocotrienol, γ-tocotrienol, δ-tocotrienol, acetate tocopherol and nicotinate tocopherol and derivatives thereof, trolox and derivatives thereof, hinokitiol, cepharanthine, α-linolenic acid, γ-linolenic acid, eicosapentaenoic acid and derivatives thereof, organic acids selected from glycolic acid, succinic acid, lactic acid and salicylic acid and derivatives thereof, estradiol and derivatives thereof, silk protein and decomposition products thereof, or derivatives thereof, hemoglobin or decomposition products thereof, lactoferrin or decomposition products thereof, animal-derived extracts such as royal jelly, placenta extract, calf blood extract solution, serum protein-free extract, spleen extract, egg ingredients, cock's crest extract, shell extract, shell fish meat extract, Mollusca extract and fish meat extract, microorganism-derived extracts such as fermentation products and metabolic products, plant extracts such as asparagus extract, apricot extract, ginkgo extract, phellodendron extract, barley extract, Panax ginseng extract, orange extract, kiwi extract, cucumber extract, shiitake extract, Equisetum arvense extract, Swertia japonica extract, Zizyphi fructus extract, Capsicum annuum extract, Calendula officinalis extract, carrot extract, garlic extract, Parthenium hysterophorus extract, grape seed extract, beech bud extract, peach extract, eucalyptus extract, Ganoderma lucidum extract, lettuce extract, lemon extract, rosemary extract and the like.

Examples of the moisturizing agent include mucopolysaccharides, proteins or decomposition products thereof and derivatives thereof, soybean or egg-derived phospholipid, glycolipid, ceramide, mucin, honey, sugars such as erythritol, maltose, maltitol, xylitol, xylose, pentaerythritol, fructose and dextrin and derivatives thereof, acidic polysaccharides such as hyaluronic acid, urea, amino acids such as asparagine, aspartic acid, alanine, arginine, isoleucine, ornithine, glutamine, glycine, glutamic acid, cysteine, cystine, citrulline, threonine, serine, tyrosine, tryptophan, theanine, valine, histidine, hydroxylysine, hydroxyproline, pyrrolidonecarboxylic acid, proline, phenylalanine, methionine and lysine and derivatives thereof, D-panthenol, whey protein, Angelica keiskei extract, avocado extract, almond extract, Althaea extract, Arnica montana extract, aloe extract, strawberry extract, Ceratonia siliqua extract, rice extract, Artemisia capillaris Thunb extract, fennel extract, turmeric extract, Malva sylvestris extract, Perilla frutescens extract, Scutellaria root extract, Coptis rhizome extract, Lamium album var. barbatum extract, Hypericum erectum extract, Ononis spinosa extract, olive oil,

seaweed extract, cacao butter, German chamomile extract, Avena fatua extract, Garcinia Cambodia extract, Glycyrrhiza glabra extract, raspberry extract, Hedera rhombea extract, Lonicera japonica extract, gardenia extract, Sasa veitchii extract, grape fruit extract, Sophora root extract, Nasturtium officinale extract, Gentiana lutea extract, Geranium thunbergii extract, Arctium lappa extract, Clematis apiifolia extract, sesame extract, wheat extract, Symphytum officinale extract, Asiasarum root extract, Cactales extract, Saponaria officinalis extract, Salvia extract, Crataegus extract, Butyrospermum parkii extract, Perilla extract, Rehmannia glutinosa extract, Spiraea extract, peony root extract, ginger extract, white birch extract, Malva sylvestris extract, Cnidium rhizome extract, Mori cortex extract, soybean extract, Thymus vulgaris extract, tea extract, camellia extract, Angelica radix extract, corn extract, plant worm extract, Houttuynia cordata Thunb extract, tormentilla extract, Lupinus extract, Ophiopogon tuber extract, parsley extract, Mentha extract, Mentha spicata extract, Mentha piperita extract, Hamamelis extract, rose extract, hinoki extract, sunflower extract, grape extract, Butchers bloom extract, prune extract, Luffa aegyptiaca extract, Tilia extract, Paeonia extract, hops extract, jojova oil, borage oil, macadamia nut extract, pine extract, Cydonia oblonga extract, Aesculus hippocastanum extract, Sapindus mukurossi extract, Lithospermum erythrorhizon extract, meadowhome oil, melissa extract, Rodgersia podophylla extract, Saxifraga stolonifera extract, Citrus junos extract, lily extract, Coix seed extract, lime extract, Momordica grosvenori extract, lavender extract, apple extract, gentian extract, Astragalus sinicus extract, Sanguisorba extract, alkali simple thermal spring, deep water and the like.

Examples of the metal chelating agent include malic acid, citric acid, salicylic acid, tartaric acid, gluconic acid, phytic acid and derivatives thereof, ethylenediaminetetraacetic acid and derivatives thereof, diethylenetriaminepentaacetic acid and derivatives thereof, N-carboxymethyl-aspartic acid and derivatives thereof, N-carboxymethyl-glutamic acid and derivatives thereof, N,N-bis(carboxymethyl)-aspartic acid and derivatives thereof, N,N-bis(carboxymethyl)-glutamic acid and derivatives thereof, N,N-bis(succinate)-ethylenediamine and derivatives thereof, desfferioxamine, o-phenanthroline, transferrin, ferritin, lactoferrin, caffeic acid, maltol, purpurogalin, pyrogallol, sodium polyphosphate, sodium metaphosphate, sodium hexametaphosphate and the like.

Examples of the oleaginous material include fats and oils such as animal and vegetable oils, waxes such as lanolin, hydrocarbons such as paraffin, higher alcohols such as cetanol, higher fatty acids such as stearic acid, sterols, phospholipids such as lecithin, synthetic esters such as myristic acid, metal soaps, silicone oil, perfluoropolymers, perfluoropolyethers and the like.

Examples of the surfactant include anionic surfactants, cationic surfactants, nonionic surfactants, emulsifiers, solubilizers and the like.

Examples of the solvent include lower alcohols such as ethanol, ethers, glycerins, liquid nonionic surfactants, liquid oleaginous materials, other organic solvents, water and the like.

Examples of the polymeric substance include polyamino acids such as polyaspartic acid, ε-polylysine, and γ-polyglutamic acid and derivatives thereof, naturally occurring polymer compounds such as collagen and elastin, semi-synthetic polymer compounds such as partially deacetylated chitin, synthetic polymer compounds such as carboxymethyl cellulose and the like.

Examples of the powdery substance include inorganic pigments such as talc, functional pigments such as synthetic mica, particulate composite powders (hybrid fine powders), pearl-gloss pigments such as titanium dioxide-coated mica, photochromic pigments, polymer powders such as nylon powder, organic powders such as N-ε-lauroyl lysine and the like.

Examples of the dye include Tar Dye Group I designated by law, Tar Dye Group II designated by law, Tar Dye Group III designated by law, hair dyes, natural dyes, mineral dyes and the like.

Examples of the fragrance include fragrance from animals such as musk, fragrance from plants such as jasmine, synthetic fragrance such as α-amylcinnamaldehyde, composite fragrance and the like.

Examples of the transdermal absorption promoting agent include urea, 2-pyrrolidone, 1-hexanol, 1-octanol, 1-decanol, 1-menthol, sodium laurylsulfate, isopropyl myristate, n-hexyl acetate, oleic acid and the like.

Examples of the steroid hormone include 21-acetoxypregnenolone, alclometasone, algestone, amcinonide, beclomethasone, betamethasone, budesonide, chloroprednisone, clobetasol, clocortolone, cloprednol, corticosterone, cortisone, cortivazol, deflazacort, desonide, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, formocortal, halcinonide, halometasone, halopredone acetate, hydrocortamate, hydrocortisone, hydrocortisone phosphate, hydrocortisone-21-sodium succinate, hydrocortisone tebutate, mazipredone, medrysone, meprednisone, methylprednisolone, mometasone furoate, paramethasone, prednicarbate, prednisolone-21-diethylaminoacetate, prednisone sodium phosphate, prednisolone sodium succinate, prednisolone sodium-21-m-sulfobenzoate, prednisolone-21-stearoylglycolate, prednisolone tebutate, prednisolone-21-trimethylacetate, preidnisone, prednival, prednylidene, prednylidene- 21-diethylaminoacetate, tixocortal, triamcinolone, triamcinolone acetonide, triamcinolone benetonide and triamcinolone hexacetonide, fluticasone and the like.

The dosage form of the cosmetic or the skin preparation for external use containing the above zinc salt of an acylamino acid is not particularly limited, and a given dosage form such as a solution, a paste, a gel, a solid or a powder

can be taken. Further, the cosmetic or the skin preparation for external use of the present invention can be used in an oil, a lotion, a cream, an emulsion, a gel, a shampoo, a hair rinse, a hair conditioner, an enamel, a foundation, a lip stick, a face powder, a facial mask, an ointment, a tablet, an injection, a granule, a capsule, a perfume, a powder, an eau de Cologne, a dental paste, a soap, an aerosol and a cleansing foam, and additionally in an agent for preventing and improving skin aging, an agent for preventing and improving dermatitis, a bathing agent, a hair growth agent, a skin essence, an agent for preventing sunburn, an agent for preventing and improving light photosensitivity such as xeroderma pigmentosum and sunlight urticaria, an agent for preventing and improving photoallergy, an agent for preventing and improving photoinhibition of immunity, or an agent for preventing and improving rough skin caused by such as injuries, cracks and chaps, a disinfectant, an antibacterial agent, an insecticide, a pest control agent, a keratolytic agent, an agent for epidermal peeling, an agent for preventing and improving acne, an agent for preventing and improving various skin diseases such as keratosis, xeroderma, ichthyosis and psoriasis and the like.

Further, other components commonly used in a cosmetic or a skin preparation for external use can be added to the cosmetic or the skin preparation for external use containing the above component of the zinc salt of an acylamino acid or the zinc salt of an organic acid in a range which does not inhibit the effect of the present invention. Examples of the other components commonly used in a cosmetic or a skin preparation for external use include a preservative, an agent for preventing browning, a buffer, an agent for acne, an agent for preventing dandruff or itching, an antiperspirant and deodorant agent, an agent for burn injury, an anti-mite and lice agent, an agent for softening keratin, an agent for xeroderma, an antiviral agent, a hormone, a vitamin, an amino acid, a peptide, a protein, an astringent agent, a freshening agent, a stimulating agent, an animal-derived component, a plant-derived component, an antibiotic, an antifungal agent, a hair growth agent and the like.

Examples

Hereinafter, the present invention will be described more specifically with reference to Examples (synthesis examples, test examples and formulation examples), however, the invention is not limited to these examples. Incidentally, in these Examples, the blending amounts are represented by % by weight.

<u>Synthesis example 1: Synthesis of zinc salt of lauroyl-L-glutamic acid, zinc salt of lauroyl glycine, zinc salt of lauroyl-L-histidine</u>

Zinc salt of lauroyl-L-glutamic acid was synthesized by the following method. Lauroyl-L-glutamic acid synthesized by a standard method (250 mg, 0.76 mmol) was dissolved in 10 ml of ethyl alcohol, and an ethanol solution of 1% zinc acetate (Wako Pure Chemical Industries, Ltd.) was prepared separately. This 1% zinc acetate (18.4 ml) (0.84 mmol of zinc acetate, 1.1 equivalent weight of lauroyl-L-glutamic acid) was added to 10 ml of the previously prepared ethanol solution of lauroyl-L-glutamic acid, and a produced precipitate was separated by filtration. The obtained precipitate was washed with water, ethanol and acetone, then, dried under reduced pressure, whereby 200 mg of zinc salt of lauroyl-L- glutamic acid was obtained. Zinc salt of lauroyl glycine and zinc salt of lauroyl-L-histidine were synthesized by reacting a lauroyl amino acid synthesized in accordance with a standard method with zinc acetate in the same manner as above, respectively. The results of elemental analysis measurement of these compounds are shown in the following Table 1.

[Table 1]

| Compound | Composition (%) | | | | | | |
|---|---|---|---|---|---|---|---|
| | | Carbon | Hydrogen | Nitrogen | Oxygen | Zinc | Ratio of lauroyl amino acid / zinc |
| Zinc salt of lauroyl-L-glutamic acid | Calculated value | 51.9 | 7.4 | 3.6 | 20.4 | 16.7 | 1.0 |
| | Measured value | 51.8 | 7.5 | 3.8 | 20.1 | 16.6 | |
| Zinc salt of lauroyl glycine | Calculated value | 58.2 | 9.0 | 4.8 | 16.6 | 11.3 | 1.9 |
| | Measured value | 58.1 | 9.0 | 5.1 | 16.3 | 12.0 | |
| Zinc salt of lauroyl-L-histidine | Calculated value | 58.6 | 8.1 | 11.4 | 13.0 | 8.9 | 1.7 |
| | Measured value | 55.0 | 8.1 | 11.2 | Not measured | 9.9 | |

Test example 1: Effect of zinc salt of acylamino acid on the activation of AP-1 by ultraviolet light

To human dermal fibroblasts which had grown to confluence in a culture plate, a test compound was added in a concentration range which does not give any damage to the fibroblasts. After 18 hours, the culture medium was replaced with a phenol red-free medium. By using Dermalei M-DMR-80 (manufactured by Toshiba Medical Supply Co. , Ltd.), the fibroblasts were irradiated with ultraviolet light (UVA: 20 J/cm2). After 4 to 5 hours, the cells were collected, and a nuclear protein was extracted by a standard method. With regard to the obtained nuclear protein, activated AP-1 was detected by a gel shift assay. By measuring the radioactivity value of the AP-1 band using a bioimaging analyzer, BAS2000 (manufactured by Fuji Film Co., Ltd.), AP-1 was quantified.

The inhibition ratio of activation of AP-1 of the test compound was calculated by the following formula.

$$\text{Inhibition ratio of activation of AP-1 (\%)} = \{1 - (A1 - A3) / (A2 - A3)\} \times 100$$

A1: radioactivity value of AP-1 band with addition of test compound
A2: radioactivity value of AP-1 band without addition of test compound
A3: radioactivity value of AP-1 band without addition of test compound and without ultraviolet irradiation

The results of the test compounds according to the present invention and the comparative compounds are shown in the following Table 2. The zinc salts of lauroyl amino acids which are test compounds exhibited a higher inhibition ratio compared with a zinc salt of an amino acid such as glycine or L-glutamic acid which is known to have an action of preventing ultraviolet light by inducing metallothionein in the skin (for example, WO 9314748) or a zinc salt of a fatty acid such as lauric acid (for example, WO 0044341). These results show that the compounds according to the present invention have a high effect of inhibiting inflammation which is not possessed by a known zinc amino acid or zinc fatty acid.

[Table 2]

| Test compound | Concentration for evaluation (μM) | Inhibition ratio (%) |
|---|---|---|
| Zinc salt of N-lauroyl glycine | 50 | 59.2 |
| Zinc salt of N-lauroyl-L- glutamic acid | 50 | 89.6 |
| Zinc salt of N-lauroyl-L-histidine | 50 | 54.8 |

[Table 3]

| Comparative compound | Concentration for evaluation (μM) | Inhibition ratio (%) |
|---|---|---|
| Zinc salt of glycine | 50 | -72.8 |
| Zinc salt of L-glutamic acid | 50 | 2.4 |
| Zinc salt of lauric acid | 50 | 9.1 |

## Claims

1. An inflammation inhibitor **characterized by** comprising one or more kinds of zinc salts of an acylamino acid derivative represented by the following general formula (I):

[Chemical 1]

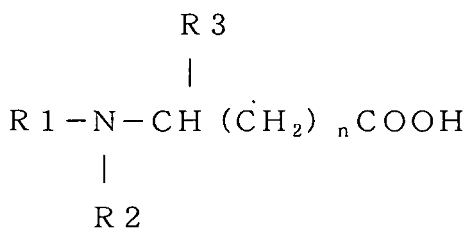

(I)

wherein, R1 represents an acyl group having 2 to 22 carbon atoms, R2 represents a hydrogen atom or a linear or branched alkyl group having 1 to 6 carbon atoms, R3 represents a side chain of valine, leucine, isoleucine, phenylalanine, methionine, tryptophan, asparagine, glutamine, serine, tyrosine, aspartic acid, glutamic acid, lysine, arginine or histidine, which is an amino acid, or a hydrogen atom, and n represents an integer of 0 or 1.

**2.** An inflammation inhibitor **characterized by** comprising one or more types of zinc salts of an acylamino acid represented by the following general formula (II):

[Chemical 2]

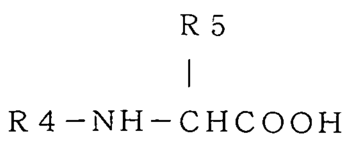

(II)

wherein, R4 represents an acyl group having 2 to 22 carbon atoms, R5 represents an amino acid side chain of valine, leucine, isoleucine, phenylalanine, methionine, tryptophan, asparagine, glutamine, serine, tyrosine, aspartic acid, glutamic acid, lysine, arginine or histidine, or a hydrogen atom.

**3.** The inflammation inhibitor according to claim 2, wherein R4 is selected from the group consisting of an octanoyl, decanoyl, lauroyl, myristoyl, palmitoyl, stearoyl and cocoyl group.

**4.** The inflammation inhibitor according to either one of claims 2 and 3, wherein R5 is selected from an amino acid side chain of glutamic acid or histidine, or a hydrogen atom.

**5.** A preventive or therapeutic agent for a skin inflammatory disease **characterized by** comprising the inflammation inhibitor according to any one of claims 1 to 4.

**6.** The preventive or therapeutic agent according to claim 5, wherein the skin inflammatory disease is a disease induced by ultraviolet light.

**7.** A cosmetic additive **characterized by** comprising the inflammation inhibitor according to any one of claims 1 to 4.

**8.** A skin preparation for external use **characterized by** comprising the inflammation inhibitor according to any one of claims 1 to 4.

**9.** A method of preventing, delaying, improving or treating a skin change due to aging or cosmetically undesirable skin change accelerated by ultraviolet light, **characterized by** applying a cosmetic or a skin preparation for external use comprising as an active ingredient, one or more kinds selected from the inflammation inhibitors according to any one of claims 1 to 4 to the skin.

## INTERNATIONAL SEARCH REPORT

International application No.
PCT/JP2005/011404

A. CLASSIFICATION OF SUBJECT MATTER
Int.Cl[7] A61K31/315, 7/00, 7/42, 7/48, 31/4172, A61P17/00, 29/00, 43/00

According to International Patent Classification (IPC) or to both national classification and IPC

B. FIELDS SEARCHED
Minimum documentation searched (classification system followed by classification symbols)
Int.Cl[7] A61K31/315, 7/00, 7/42, 7/48, 31/4172, A61P17/00, 29/00, 43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAplus(STN), MEDLINE(STN), EMBASE(STN), BIOSIS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2000-517335 A (SOCIETE D'EXPLOITATION DE PRODUITS POUR LES INDUSTRIES CHIMIQUES, S.E.P.P.I.C.), 26 December, 2000 (26.12.00), Full text & WO 1998/009611 A1 & EP 925058 A1 | 1-8 |
| A | JP 6-256274 A (Laboratoires Phytocos), 13 September, 1994 (13.09.94), Full text & EP 601911 A1 & US 5504228 A | 1-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 September, 2005 (16.09.05) | 11 October, 2005 (11.10.05) |
| Name and mailing address of the ISA/ Japanese Patent Office | Authorized officer |
| Facsimile No. | Telephone No. |

Form PCT/ISA/210 (second sheet) (January 2004)

INTERNATIONAL SEARCH REPORT

International application No. PCT/JP2005/011404

C (Continuation). DOCUMENTS CONSIDERED TO BE RELEVANT

| Category* | Citation of document, with indication, where appropriate, of the relevant passages | Relevant to claim No. |
|---|---|---|
| A | WO 1993/014748 A1 (Otsuka Pharmaceutical Co., Ltd.), 05 August, 1993 (05.08.93), Full text & JP 5-513101 A & EP 583479 A1 & US 5582817 A | 1-8 |
| A | JP 2002-179518 A (Kyowa Hakko Kogyo Co., Ltd.), 26 June, 2002 (26.06.02), Full text & US 2002/122784 A1 | 1-8 |
| A | JP 11-343235 A (Ajinomoto Co., Inc.), 14 December, 1999 (14.12.99), Full text & EP 955046 A2 & US 6552061 B1 | 1-8 |
| A | JP 6-305906 A (Kabushiki Kaisha Nikko), 01 November, 1994 (01.11.94), Full text & KR 339776 B | 1-8 |
| A | JP 3-200879 A (Kanebo, Ltd.), 02 September, 1991 (02.09.91), Full text (Family: none) | 1-8 |
| A | JP 2-256609 A (Kenji ICHIKAWA), 17 October, 1990 (17.10.90), Full text (Family: none) | 1-8 |

Form PCT/ISA/210 (continuation of second sheet) (January 2004)

INTERNATIONAL SEARCH REPORT

International application No.
PCT/JP2005/011404

**Box No. II Observations where certain claims were found unsearchable (Continuation of item 2 of first sheet)**

This international search report has not been established in respect of certain claims under Article 17(2)(a) for the following reasons:

1. [X] Claims Nos.: 9
because they relate to subject matter not required to be searched by this Authority, namely:
The invention as set forth in claim 9 pertains to methods for treatment of the human body by therapy and thus relates to a subject matter which this International Searching Authority is not required, under the provisions of Article 17(2)(a)(i) of the PCT (continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

INTERNATIONAL SEARCH REPORT

International application No.
PCT/JP2005/011404

Continuation of Box No.II-1 of continuation of first sheet(2)

and Rule 39.1(iv) of the Regulations under the PCT, to search.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0044341 A
- WO 9314748 A
- WO 0021925 A
- US 5594012 A


### Non-patent literature cited in the description


- *Nature,* 1996, vol. 379, 335-339
- *Free Rad. Biol. Med.,* vol. 26, 174-183
- *FEBS Letters,* 1996, vol. 384, 92-96
- *Eur. Acad. Dermatol. Veneol.,* 2001, vol. 15 (1), 5-11
- *J Am. Clloge Nutrt.,* 1997, vol. 16 (5), 411-417